# EUROPÄISCHE PATENTSCHRIFT

(11) **EP 3 403 684 B1**
(45) Veröffentlichungstag und Bekanntmachung des Hinweises auf die Patenterteilung: **12.03.2025**
(21) Anmeldenummer: 18170645.8
(22) Anmeldetag: 03.05.2018
(51) Int. Cl.: A61M 25/00, A61M 25/01

(54) **ZENTRALVENÖSE KATHETERANORDNUNG**
CENTRAL VENOUS CATHETER ASSEMBLY
ENSEMBLE CATHÉTER VEINEUX CENTRAL

(30) Priorität: 17.05.2017 DE 102017208332
(43) Veröffentlichungstag der Anmeldung: 21.11.2018
(73) Patentinhaber: B. Braun Melsungen AG, 34212 Melsungen (DE)
(72) Erfinder: WEIß, André, 34302 Guxhagen (DE); SCHRÖDER, Tobias, 34212 Melsungen (DE)
(74) Vertreter: Patentanwälte Ruff, Wilhelm, Beier, Dauster & Partner mbB

(56) Entgegenhaltungen:
- EP-A1- 0 422 631
- WO-A1-2005/089851
- DE-A1- 102007 029 229
- US-A1- 2010 331 823

## Beschreibung

Die Erfindung betrifft eine zentralvenöse Katheteranordnung nach dem Oberbegriff von Anspruch 1.

Zentralvenöse Katheteranordnungen sind im Bereich der Medizintechnik allgemein bekannt. Zentralvenöse Katheteranordnungen werden auch als Zentralvenenkatheter oder zentralvenöse Zugänge bezeichnet und sind für die Verwendung bei der Infusionstherapie vorgesehen. Üblicherweise ist als Katheterschlauch eine dünne und flexible Kunststoffschlauchleitung vorgesehen, an deren distalen Schlauchende eine Katheterspitze einstückig angeformt ist. Zum Anlegen des zentralvenösen Zugangs wird das distale Schlauchende des Katheterschlauchs auf eine im Bereich der Medizintechnik allgemein bekannte Weise in eine Vene des Patienten eingeführt und in Richtung des Herzens vorgeschoben. Die Katheterspitze wird hierbei üblicherweise im Bereich des rechten Herzvorhofs des Patienten positioniert. Das proximale Schlauchende verbleibt nach Anlegen des Zugangs auf der Körperaußenseite des Patienten und ist bei bekannten Zentralvenenkathetern über ein Fixierelement fluidleitend mit mindestens einer Katheterzuleitung verbunden. Diese Verbindung zwischen dem Katheterschlauch und dem Fixierelement und/oder der Katheterzuleitung ist üblicherweise fest ausgeführt im Sinne von einstückig und/oder nicht zerstörungsfrei lösbar. Je nach medizinischer Indikation wird der zentralvenöse Zugang über unterschiedliche Venen bzw. über unterschiedliche Eintrittsstellen am Körper eines Patienten angelegt. Insoweit muss die Länge des Katheterschlauchs in Abhängigkeit von der medizinischen Indikation und/oder der Körpergröße des jeweiligen Patienten gewählt werden. Bekannte zentralvenöse Katheteranordnungen sind deshalb in unterschiedlichen Ausführungen betreffend die Schlauchlänge zwischen dem proximalen und dem distalen Schlauchende verfügbar.

Aus der US 2010/0331823 A1 ist eine Katheteranordnung mit einem Katheterschlauch und einer Verbindungsvorrichtung bekannt, mittels derer ein proximales Schlauchende des Katheterschlauchs mit einer Katheterzuleitung verbindbar ist. Die Verbindungsvorrichtung erlaubt zudem ein proximales Kürzen des Katheterschlauchs. Abgesehen davon weist die Verbindungsvorrichtung einen vergleichsweise aufwendigen Aufbau auf. Das proximale Schlauchende ist nicht ohne Weiteres zugänglich.

Aus der EP 0 422 631 A1 ist eine weitere Katheteranordnung mit einem Schlauch und einem Adapter bekannt. Der Adapter fungiert als eine Art Verbindungsvorrichtung und ist zwischen einer Freigabe- und einer Verriegelungsposition schwenkbeweglich. Dabei ist der Schlauch in der Verriegelungsposition an dem Adapter gehalten und in der Freigabeposition freigegeben.

Im Übrigen ist aus der WO 2005/089851 A1 ein Verbindungssystem für einen proximal trimmbaren Katheter bekannt. Das Verbindungssystem weist eine Muffe mit einem Lumen zur Aufnahme des Katheters auf. Zudem ist eine Gabelanordnung vorgesehen, die endseitig auf die Muffe aufsteckbar ist.

Ferner ist aus der DE 10 2007 029 229 A1 ein Spannadapter für einen Katheter bekannt. Bei dem Spannadapter wird das proximale Ende des Katheters in einer weichelastischen Spannbuchse aufgenommen und durch ein axiales Stauchen der Spannbuchse fixiert. Zum Stauchen der Spannbuchse dient ein Spannhebel, der um eine zur Längsachse des Spannadapters quer verlaufende Schwenkachse schwenkbar ist.

Aufgabe der Erfindung ist es, eine zentralvenöse Katheteranordnung der eingangs genannten Art zu schaffen, die eine universelle Verwendung erlaubt.

Diese Aufgabe wird durch eine zentralvenöse Katheteranordnung mit der Merkmalskombination des Anspruch 1 gelöst. Durch die erfindungsgemäße Lösung ist es möglich, die Länge des Katheterschlauchs auf einfache Weise an die jeweiligen Erfordernisse anzupassen. Derart kann auf die Herstellung und Lagerhaltung von Katheteranordnungen mit unterschiedlichen Schlauchlängen verzichtet werden. Zudem wird in vorteilhafter Weise ein Einkürzen der Schlauchlänge ausgehend von dem proximalen Schlauchende erreicht. Zwar ist auch ein Einkürzen ausgehend von dem distalen Schlauchende möglich. Hierbei wird jedoch die Katheterspitze abgetrennt und die Katheteranordnung unter Umständen unbrauchbar. Die mindestens eine Katheterzuleitung kann ein Lumen zur Zuleitung einer Medikamentenflüssigkeit sein, das an seinem proximalen Ende einem Katheteransatz aufweist, der insbesondere ein als solcher bekannter Luer-Lock-Anschluss sein kann. Im Sinne der Erfindung bedeutet "distal" eine Orientierung zum Körperzentrum hin und "proximal" eine Orientierung vom Körperzentrum hinweg. Unter "kürzbar" oder "einkürzbar" im Sinne der Erfindung wird eine Änderung der Länge zwischen dem proximalen und dem distalen Schlauchende verstanden, die jedoch nicht zwingend mit einem Abtrennen eines Schlauchabschnitts einhergehen muss. Beispielsweise kann auch ein Anordnen eines Schlauchabschnitts in Gestalt einer Schlinge, einer Schlaufe oder dergleichen eine Änderung der Länge zwischen den Schlauchenden und somit ein Kürzen im Sinne der Erfindung bewirken. Mit "lösbar" im Sinne der Erfindung ist ein zerstörungsfreie Trennbarkeit gemeint.

Vorteilhafterweise umfasst der Begriff "lösbar" im Sinne der Erfindung eine ohne Weiteres herstellbare Wiederverbindbarkeit nach einem vorherigen zerstörungsfreien Trennen.

Weiter gemäß der Erfindung ist die Verriegelungseinheit relativ zu dem Gehäuse von einer Verriegelungsposition in eine Freigabeposition bewegbar, wobei das proximale Schlauchende in der Verriegelungsposition in einer Wirkverbindung mit der Verriegelungseinheit steht und mittels dieser an dem Gehäuse festgelegt ist und wobei das proximale Schlauchende in der Freigabeposition von dem Gehäuse lösbar ist. Die Verriegelungseinheit weist ein Klemmelement auf, das in der Verriegelungsposition mit einem Mantelabschnitt des proximalen Schlauchendes in einer lösbaren Klemmverbindung steht. Derart wird eine besonders zuverlässige und im Bedarfsfall leicht lösbare Verbindung des proximalen Schlauchendes an der Verbindungsvorrichtung erreicht. Demzufolge ergibt diese Ausgestalt der Erfindung eine besonders einfach und zuverlässig handhabbare Katheteranordnung.

Weiter gemäß der Erfindung gibt die Verriegelungseinheit in der Freigabeposition eine Gehäuseöffnung frei, durch welche zumindest ein Abschnitt des proximalen Schlauchendes aus dem Gehäuse entnehmbar ist. In der Verriegelungsposition ist die Gehäuseöffnung von der Verriegelungseinheit zumindest abschnittsweise bedeckt. Vorteilhaftweise ist demnach zumindest ein Abschnitt des proximalen Schlauchendes in dem Gehäuse angeordnet. Die Gehäuseöffnung ist im Wesentlichen radial zu der Mantelfläche des proximalen Schlauchendes ausgerichtet ist. Derart wird eine gute Zugänglichkeit und manuelle Entnahme des proximalen Schlauchendes aus dem Gehäuse erreicht.

Weiter gemäß der Erfindung ist die Verriegelungseinheit relativ zu dem Gehäuse verschwenkbar an diesem befestigt. Vorteilhafterweise ist die Verriegelungseinheit um eine quer zur Axialrichtung des proximalen Schlauchendes orientierte Schwenkachse verschwenkbar. Eine besonders kostengünstige Fertigung wird erreicht, wenn die Verriegelungseinheit mittels eines Filmscharniers an dem Gehäuse befestigt ist.

In weiterer Ausgestaltung der Erfindung weist das Gehäuse einen im Wesentlichen koaxial zu dem proximalen Schlauchende erstreckten zylindrischen Gehäuseabschnitt auf, der das proximale Schlauchende zumindest abschnittsweise radial umgreift. Der zylindrische Gehäuseabschnitt bildet einen in Richtung des proximalen Schlauchabschnitts erstreckten Fortsatz des Gehäuses. Durch die radiale Führung des proximalen Schlauchendes wird einem ungewollten Abknicken des Katheterschlauchs im Bereich des Übergangs zwischen der Verbindungsvorrichtung und dem sich daran anschließenden Schlauchverlauf vorteilhaft entgegengewirkt.

In weiterer Ausgestaltung der Erfindung weist der zylindrische Gehäuseabschnitt an seiner distalen, das heißt der Körpereintrittsstelle zugewandten, Stirnseite einen Konus auf, der zur Anlage an einer Körpereintrittsstelle des Katheterschlauchs ausgebildet ist. Der Konus ermöglicht eine für den Patienten weniger störende Anlage der Katheteranordnung an der betreffenden Eintrittsstelle des Katheterschlauchs in den Körper des Patienten. Zudem beugt die konusförmige Gestaltung vorteilhafterweise Blutungen im Bereich der Eintrittsstelle vor.

In weiterer Ausgestaltung der Erfindung ist der zylindrische Gehäuseabschnitt durch ein Schlauchelement gebildet, dessen Innendurchmesser im Wesentlichen dem Außendurchmesser des Katheterschlauchs entspricht, so dass dieser in einer Freigabeposition der Verriegelungseinheit axial bewegbar in dem Schlauchelement geführt ist. Vorteilhafterweise ist das Schlauchelement derart flexibel gestaltet, dass einerseits eine komfortable Anlage des zylindrischen Gehäuseabschnitts an der Eintrittsstelle und andererseits eine ausreichende radiale Stützung des proximalen Schlauchendes gegen ein ungewolltes Abknicken erreicht ist.

In weiterer Ausgestaltung der Erfindung weist die Verbindungsvorrichtung mindestens ein Anschlusselement auf, an dem mindestens eine Katheterzuleitung anschließbar und derart fluidleitend mit dem Katheterschlauch verbindbar ist. Das mindestens eine Anschlusselement ist vorteilhafterweise an dem proximalen Stirnendbereich des Gehäuses angeordnet. Vorteilhafterweise ist das Anschlusselement ein im Bereich der Medizintechnik als solcher bekannter Luer-Lock-Anschluss.

In weiterer Ausgestaltung der Erfindung ist die Katheterspitze atraumatisch ausgestaltet, insbesondere als Softspitze. Atraumatische Katheterspitzen sind im Bereich der Medizintechnik als solche allgemein bekannt. Insoweit ist die atraumatische Katheterspitze derart, insbesondere weich und/oder flexibel, ausgestaltet, dass eine Verletzung der Vene beim Vorschieben des distalen Katheterschlauchs vermieden wird.

Weitere Vorteile und Merkmale der Erfindung ergeben sich aus den Ansprüchen sowie aus der nachfolgenden Beschreibung eines bevorzugten Ausführungsbeispiels der Erfindung das anhand der Zeichnungen dargestellt ist.
- Fig. 1: zeigt in schematischer Seitenansicht eine Ausführungsform einer erfindungsgemäßen zentralvenösen Katheteranordnung in einer Verriegelungsposition und
- Fig. 2: die Katheteranordnung nach Fig. 1 in einer Freigabeposition.

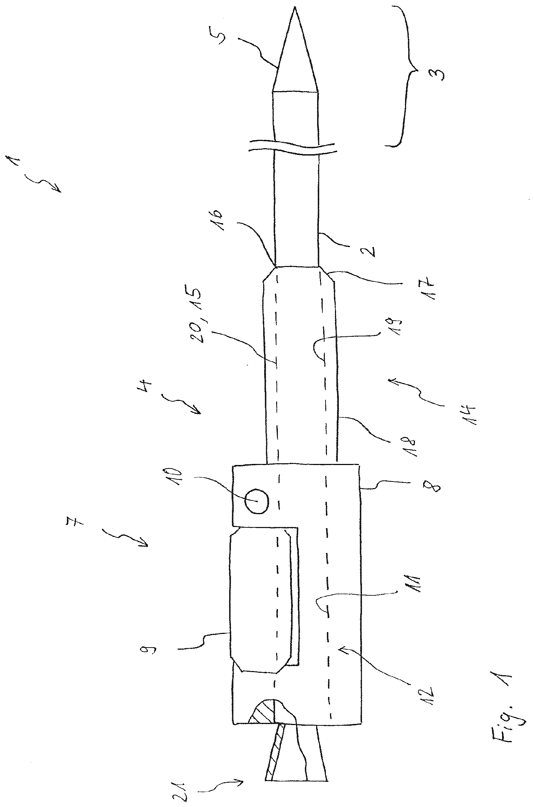

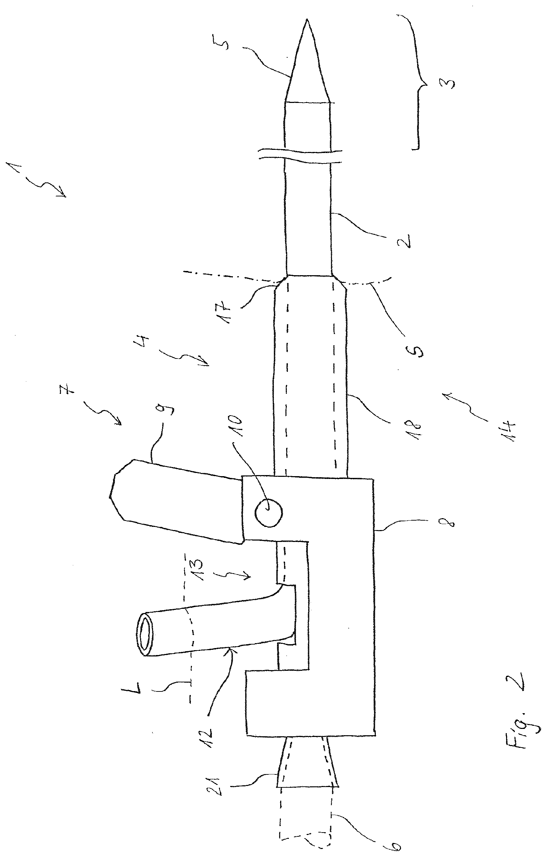

Eine zentralvenöse Katheteranordnung 1 nach den Fig. 1 und 2 ist für die Verwendung bei einer Infusionstherapie vorgesehen. Die Katheteranordnung 1 weist einen Katheterschlauch 2 zum Einführen in eine Vene eines Patienten mit einem distalen Schlauchende 3 und einem proximalen Schlauchende 4 auf. An dem distalen Schlauchende 3 ist eine spezifisch ausgeformte Katheterspitze 5 einstückig angeformt. Zum Anlegen eines zentralen Zugangs zu dem Venensystem des Patienten wird das distale Schlauchende 3 auf eine im Bereich der Medizintechnik allgemein bekannte Weise ausgehend von einer Körpereintrittsstelle in eine Vene des Patienten eingeführt und der Katheterschlauch 2 wird in der Vene vorgeschoben, bis die Katheterspitze 5 eine gewünschte Position erreicht hat. Insbesondere zur Verbindung des proximalen Schlauchendes 4 mit mindestens einer, in Fig. 2 strichliert angedeuteten Katheterzuleitung 6 ist eine Verbindungsvorrichtung 7 mit einem Gehäuse 8 und einer Verriegelungseinheit 9 vorgesehen. Das proximale Schlauchende 4 ist auf eine im Folgenden näher beschriebene Weise lösbar an der Verbindungsvorrichtung 7 festgelegt.

Die Verriegelungseinheit 9 der Verbindungsvorrichtung 7 ist relativ zu dem Gehäuse 8 verschwenkbar an diesem befestigt. Zu diesem Zweck weist die Verbindungsvorrichtung 7 eine Schwenkachse 10 auf, die senkrecht zu einer Haupterstreckungsrichtung der Verbindungsvorrichtung 7 orientiert ist. Die Verriegelungseinheit 9 ist relativ zu dem Gehäuse 8 um die Schwenkachse 10 zwischen einer Verriegelungsposition V (Fig. 1) und einer Freigabeposition F (Fig. 2) verlagerbar. Das proximale Schlauchende 4 erstreckt sich zumindest abschnittsweise entlang einer Innenwandung 11 der Verbindungsvorrichtung 7. In der Verriegelungsposition V ist das proximale Schlauchende 4 in einer Wirkverbindung mit der Verriegelungseinheit 9 und auf diese Weise an dem Gehäuse 8 der Verbindungsvorrichtung 7 festgelegt. Die Verriegelungseinheit 9 ist als Klemmelement gestaltet, welches in der Verriegelungsposition V radial auf einen Schlauchmantelabschnitt 12 des proximalen Schlauchendes 4 einwirkt und dieses derart an der Innenwandung 11 festlegt. In der Freigabeposition F ist die Wirkverbindung zwischen dem proximalen Schlauchende 4 bzw. zwischen dem Schlauchmantelabschnitt 12 und der Verriegelungseinheit 9 aufgehoben, so dass das proximale Schlauchende 4 von dem Gehäuse 8 der Verbindungsvorrichtung 7 lösbar ist.

Wie anhand Fig. 2 ersichtlich ist, weist das Gehäuse 8 eine Gehäuseöffnung 13 auf, welche von der Verriegelungseinheit 9 in der Freigabeposition F freigegeben und in der Verriegelungsposition V bedeckt ist. Insoweit ist in der Freigabeposition F zumindest ein Abschnitt des proximalen Schlauchendes 4 durch die Gehäuseöffnung 13 zugänglich und aus dem Gehäuse 8 entnehmbar. Die Gehäuseöffnung 13 ist im Wesentlichen radial zu dem proximalen Schlauchendes 4 orientiert.

Das Gehäuse 8 der Verbindungsvorrichtung 7 weist einen im Wesentlichen koaxial zu dem proximalen Schlauchende 4 erstreckten zylindrischen Gehäuseabschnitt 14 auf. Der zylindrische Gehäuseabschnitt 14 umgreift einen zweiten Schlauchmantelabschnitt 15 des proximalen Schlauchendes 4 radial und weist an seiner distalen Stirnseite 16 einen Konus 17 auf. Der Konus 17 ist in angelegtem Zustand der Katheteranordnung 1 zur Anlage an einer nicht näher bezeichneten und anhand Fig. 2 lediglich strichliert angedeuteten Körpereintrittsstelle S ausgebildet. Der zylindrische Gehäuseabschnitt 14 ist durch ein Schlauchelement 18 gebildet, dessen Innendurchmesser 19 im Wesentlichen dem Außendurchmesser 20 des zweiten Schlauchmantelabschnitts 15 des Katheterschlauchs 2 entspricht. Derart ist das proximale Schlauchende 4 in dem Schlauchelement 18 axial bewegbar geführt, sobald die Verriegelungseinheit 9 ihre Freigabeposition F einnimmt.

Zudem weist die Verbindungsvorrichtung 7 ein Anschlusselement 21 auf, das als Luer-Lock-Anschluss ausgebildet ist. Das Anschlusselement 21 ist an dem proximalen Stirnende des Gehäuses 8 angeordnet und fluidleitend mit der Katheterzuleitung 6 verbindbar. Über das Anschlusselement 21 kann demzufolge eine fluidleitende Verbindung zwischen der Katheterzuleitung 6 und dem Stirnendbereich des proximalen Schlauchendes 4 hergestellt werden.

Im Übrigen weist die Katheteranordnung 1 eine an sich bekannte Softspitze als Katheterspitze 5 auf, die atraumatisch gestaltet ist, so dass eine Verletzung der Vene beim Vorschub des distalen Schlauchendes 3 durch die Katheterspitze 5 vermieden wird.

Zum Erreichen einer ausgewählten Schlauchlänge zwischen dem proximalen Schlauchende 4 und dem distalen Schlauchende 3 wird der Katheterschlauch 2 ausgehend von dem proximalen Schlauchende 4 eingekürzt. Ausgehend von dem anhand Fig. 1 ersichtlichen Zustand der Katheteranordnung 1 wird zu diesem Zweck zunächst die Verriegelungseinheit 9 von der Verriegelungsposition V in die Freigabeposition F bewegt. Derart wird die Klemmverbindung zwischen der Verriegelungseinheit 9 und dem Schlauchmantelabschnitt 12 des proximalen Schlauchendes 4 gelöst. Zudem wird durch das Verschwenken der Verriegelungseinheit 9 von der Verriegelungsposition V in die Freigabeposition F die Gehäuseöffnung 13 freigegeben. Derart ist ein Abschnitt des proximalen Schlauchendes 4 durch die Gehäuseöffnung 13 zugänglich und insbesondere manuell aus dem Gehäuse 8 entnehmbar, wie anhand Fig. 2 schematisch angedeutet ist. Da der zweite Schlauchmantelabschnitt 15 des proximalen Schlauchendes 4 axial bewegbar in dem durch das Schlauchelement 18 gebildeten zylindrischen Gehäuseabschnitt 14 geführt ist, kann der durch die Verriegelungseinheit 9 freigegebene Abschnitt des proximalen Schlauchendes 4 aus der Gehäuseöffnung 13 herausgezogen werden. Das Kürzen der Länge des Katheterschlauchs 2 kann durch Abtrennen eines Abschnitts des proximalen Schlauchendes 4 entlang einer anhand Fig. 2 ersichtlichen strichliert dargestellten Schnittlinie L erfolgen. Das derart eingekürzte proximale Schlauchende 4 kann durch eine in distale Richtung weisende Axialbewegung des Katheterschlauchs 2 durch die Gehäuseöffnung 13 in das Gehäuse 8 eingezogen und darauffolgend durch ein Verschwenken der Verriegelungseinheit 9 von der Freigabeposition F in die Verriegelungsposition V an der Verbindungsvorrichtung 7 festgelegt werden.

## Patentansprüche

1. Zentralvenöse Katheteranordnung (1) zum Anlegen eines zentralen Zugangs zu einem Venensystem eines Patienten aufweisend einen Katheterschlauch (2) zum Einführen in eine Vene des Venensystems mit einem eine Katheterspitze (5) aufweisenden distalen Schlauchende (3) sowie einem proximalen Schlauchende (4), das fluidleitend mit mindestens einer Katheterzuleitung (6) verbindbar ist,
wobei eine Verbindungsvorrichtung (7) mit einem Gehäuse (8) und einer Verriegelungseinheit (9) vorgesehen ist, wobei das proximale Schlauchende (4) derart lösbar an der Verbindungsvorrichtung (7) festgelegt ist, dass der Katheterschlauch (2) ausgehend von dem proximalen Schlauchende (4) kürzbar ist zum Erreichen einer ausgewählten Schlauchlänge zwischen dem proximalen (4) und dem distalen Schlauchende (3), und wobei das proximale Schlauchende (4) mittels der Verbindungsvorrichtung (7) fluidleitend mit der mindestens einen Katheterzuleitung (6) verbindbar ist, und dass die Verriegelungseinheit (9) ein Klemmelement aufweist, das relativ zu dem Gehäuse (8) von einer Verriegelungsposition (V) in eine Freigabeposition (F) verschwenkbar an dem Gehäuse (8) befestigt ist, und das in der Verriegelungsposition (V) derart radial auf einen Schlauchmantelabschnitt (12) des proximalen Schlauchendes (4) einwirkt, dass das proximale Schlauchende (4) mittels des Klemmelements an dem Gehäuse (8) festgelegt ist, wobei das proximale Schlauchende (4) in der Freigabeposition (F) von dem Gehäuse (8) lösbar ist, **dadurch gekennzeichnet, dass** die Verriegelungseinheit (9) in der Freigabeposition (F) eine Gehäuseöffnung (13) freigibt, welche im Wesentlichen radial zu der Mantelfläche des proximalen Schlauchendes (4) ausgerichtet ist und durch welche zumindest ein Abschnitt des proximalen Schlauchendes (4) aus dem Gehäuse (8) entnehmbar ist.

2. Zentralvenöse Katheteranordnung (1) nach einem der vorhergehenden Ansprüche, **dadurch gekennzeichnet, dass** das Gehäuse (8) einen im Wesentlichen koaxial zu dem proximalen Schlauchende (4) erstreckten zylindrischen Gehäuseabschnitt (14) aufweist, der das proximale Schlauchende (4) zumindest abschnittsweise radial umgreift.

3. Zentralvenöse Katheteranordnung (1) nach Anspruch 2, **dadurch gekennzeichnet, dass** der zylindrische Gehäuseabschnitt (14) an seiner distalen Stirnseite (16) einen Konus (17) aufweist, der zur Anlage an einer Körpereintrittsstelle des Katheterschlauchs (2) ausgebildet ist.

4. Zentralvenöse Katheteranordnung (1) nach Anspruch 2 oder 3, **dadurch gekennzeichnet, dass** der zylindrische Gehäuseabschnitt (14) durch ein Schlauchelement (18) gebildet ist, dessen Innendurchmesser (19) im Wesentlichen dem Außendurchmesser (20) des Katheterschlauchs (2) entspricht, so dass dieser in einer Freigabeposition (F) der Verriegelungseinheit (9) axial bewegbar in dem Schlauchelement (18) geführt ist.

5. Zentralvenöse Katheteranordnung (1) nach einem der vorhergehenden Ansprüche, **dadurch gekennzeichnet, dass** die Verbindungsvorrichtung (9) mindestens ein Anschlusselement (21) aufweist, an dem die mindestens eine Katheterzuleitung (6) anschließbar und derart fluidleitend mit dem Katheterschlauch (2) verbindbar ist.

6. Zentralvenöse Katheteranordnung (1) nach einem der vorhergehenden Ansprüche, **dadurch gekennzeichnet, dass** die Katheterspitze (5) atraumatisch ausgestaltet ist, insbesondere als Softspitze.

## Claims

1. Central venous catheter assembly (1) for applying a central access to a venous system of a patient, having a catheter tube (2) for inserting into a vein of the venous system, with a distal tube end (3) having a catheter tip (5), and with a proximal tube end (4), which can be connected in a fluid-conducting manner to at least one catheter supply line (6),
wherein a connecting device (7) with a housing (8) and with a locking unit (9) is provided, wherein the proximal tube end (4) is fastened releasably to the connecting device (7) in such a way that the catheter tube (2), starting from the proximal tube end (4), can be shortened in order to obtain a selected tube length between the proximal tube end (4) and the distal tube end (3), and wherein the proximal tube end (4) can be connected by means of the connecting device (7) to the at least one catheter supply line (6) in a fluid-conducting manner, and the locking unit (9) has a clamping element which is fastened to the housing (8) so as to be pivotable relative to the housing (8) from a locking position (V) to a release position (F), and which in the locking position (V) acts radially on a tube jacket portion (12) of the proximal tube end (4) in such a way that the proximal tube end (4) is fastened to the housing (8) by means of the clamping element, wherein the proximal tube end (4) in the release position (F) is releasable from the housing (8), **characterized in that** the locking unit (9) in the release position (F) frees a housing opening (13), which is oriented substantially radially with respect to the jacket surface of the proximal tube end (4) and through which at least a portion of the proximal tube end (4) is removable from the housing (8).

2. Central venous catheter assembly (1) according to any one of the preceding claims, **characterized in that** the housing (8) has a cylindrical housing portion (14) which extends substantially coaxially to the proximal tube end (4) and which engages radially around the proximal tube end (4) at least in sections.

3. Central venous catheter assembly (1) according to Claim 2, **characterized in that** the cylindrical housing portion (14) has, on its distal end face (16), a cone (17) which is designed to bear on a point of entry of the catheter tube (2) into the body.

4. Central venous catheter assembly (1) according to Claim 2 or 3, **characterized in that** the cylindrical housing portion (14) is formed by a tube element (18), the internal diameter (19) of which substantially corresponds to the external diameter (20) of the catheter tube (2), so that the latter is guided axially movably in the tube element (18) in a release position (F) of the locking unit (9).

5. Central venous catheter assembly (1) according to any one of the preceding claims, **characterized in that** the connecting device (9) has at least one connector element (21), to which the at least one catheter supply line (6) can be attached and can be connected in a fluid-conducting manner to the catheter tube (2).

6. Central venous catheter assembly (1) according to any one of the preceding claims, **characterized in that** the catheter tip (5) is designed to be atraumatic, in particular as a soft tip.

## Revendications

1. Agencement de cathéter veineux central (1) destiné à fournir un accès central à un système veineux d'un patient, présentant un tube de cathéter (2) destiné à être inséré dans une veine du système veineux, avec une extrémité de tube distale (3) présentant une pointe de cathéter (5) ainsi qu'une extrémité de tube proximale (4) qui peut être reliée de manière fluidique à au moins une conduite d'alimentation de cathéter (6),
il étant prévu un dispositif de liaison (7) avec un boîtier (8) et une unité de verrouillage (9), l'extrémité de tube proximale (4) étant fixée de manière amovible au dispositif de liaison (7) de telle sorte que le tube de cathéter (2) peut être raccourci à partir de l'extrémité de tube proximale (4) pour atteindre une longueur de tube choisie entre l'extrémité de tube proximale (4) et l'extrémité de tube distale (3), et l'extrémité de tube proximale (4) pouvant être reliée de manière fluidique à l'au moins une conduite d'alimentation de cathéter (6) au moyen du dispositif de liaison (7), et l'unité de verrouillage (9) présentant un élément de serrage qui est fixé au boîtier (8) de manière à pouvoir pivoter par rapport au boîtier (8) d'une position de verrouillage (V) à une position de libération (F), et qui, dans la position de verrouillage (V), agit radialement sur une section de gaine de tube (12) de l'extrémité de tube proximale (4) de telle sorte que l'extrémité de tube proximale (4) est fixée au boîtier (8) au moyen de l'élément de serrage, l'extrémité de tube proximale (4) pouvant être détachée du boîtier (8) dans la position de libération (F), **caractérisé en ce que** l'unité de verrouillage (9) libère, dans la position de libération (F), une ouverture de boîtier (13) qui est orientée essentiellement radialement par rapport à la surface de gaine de l'extrémité de tube proximale (4) et à travers laquelle au moins une section de l'extrémité de tube proximale (4) peut être retirée du boîtier (8).

2. Agencement de cathéter veineux central (1) selon l'une quelconque des revendications précédentes, **caractérisé en ce que** le boîtier (8) présente une section de boîtier cylindrique (14) s'étendant essentiellement coaxialement à l'extrémité de tube proximale (4), qui entoure radialement l'extrémité de tube proximale (4) au moins par sections.

3. Agencement de cathéter veineux central (1) selon la revendication 2, **caractérisé en ce que** la section de boîtier cylindrique (14) présente sur son côté frontal distal (16) un cône (17) qui est conçu pour s'appuyer sur un point d'entrée du corps du tube de cathéter (2).

4. Agencement de cathéter veineux central (1) selon la revendication 2 ou 3, **caractérisé en ce que** la section de boîtier cylindrique (14) est formée par un élément de tube (18) dont le diamètre intérieur (19) correspond essentiellement au diamètre extérieur (20) du tube de cathéter (2), de telle sorte que celui-ci est guidé de manière mobile axialement dans l'élément de tube (18) dans une position de libération (F) de l'unité de verrouillage (9).

5. Agencement de cathéter veineux central (1) selon l'une quelconque des revendications précédentes, **caractérisé en ce que** le dispositif de liaison (9) présente au moins un élément de raccordement (21) auquel l'au moins une conduite d'alimentation de cathéter (6) peut être raccordée et peut être reliée de manière fluidique au tube de cathéter (2).

6. Agencement de cathéter veineux central (1) selon l'une quelconque des revendications précédentes, **caractérisé en ce que** la pointe de cathéter (5) est conçue de manière atraumatique, notamment comme une pointe souple.
